# DEMANDE DE BREVET EUROPEEN

(11) **EP 4 159 196 A1**
(43) Date de publication de la demande: **05.04.2023**
(21) Numéro de dépôt: 22198657.3
(22) Date de dépôt: 29.09.2022
(51) Int. Cl.: A61K 9/00, A61K 9/06, A61K 31/00, A61K 47/10, A61K 47/38, A61K 31/135

(54) **COMPOSITION PHARMACEUTIQUE TOPIQUE SOUS FORME DE GEL COMPRENANT AU MOINS DE L AMITRIPTYLINE POUR SON UTILISATION DANS LE TRAITEMENT DES DOULEURS NEUROPATHIQUES DU MEMBRE FANTÔME**

(30) Priorité: 01.10.2021 FR 2110426
(71) Demandeur: Algotherapeutix, 92150 Suresnes (FR)
(72) Inventeur: PRINCIPE NICOLAS, Paola, 78720 CERNAY LA VILLE (FR); LALLEMAND, Frédéric, 78120 RAMBOUILLET (FR); GRECO, Céline, 75012 PARIS (FR); THIROLOIX, Stéphane, 92150 SURESNES (FR)
(74) Mandataire: Casalonga

(57) **Abrégé**

La présente invention concerne une composition pharmaceutique topique sous forme de gel aqueux comprenant de 10 à 30% d'amitriptyline en poids pour son utilisation dans le traitement des douleurs du membre fantôme.

## Description

La présente invention concerne une composition pharmaceutique topique comprenant de l'amitriptyline pour son utilisation dans le traitement des douleurs du membre fantôme.

Les douleurs neuropathiques du membre fantôme (« phantom limb pain » en langue anglaise) sont des douleurs apparaissant à la suite de l'amputation ou de la désafférentation d'un membre, tel que par exemple une main, un avant-bras ou une jambe.

Ces douleurs sont considérées comme un phénomène d'algohallucinose, associé à des sensations douloureuses ou désagréables (décharges électriques, piqures, brûlures, crampes, ...) dans la répartition du membre perdu ou désafférenté. Ces douleurs peuvent être localisées sur tout le membre ou seulement sur une région du membre manquant.

Les douleurs du membre fantôme surviennent généralement dans les six mois suivant la perte d'un membre. Elles sont ressenties par environ 60 à 80% des personnes amputées d'un membre, et peuvent persister pendant des années après l'amputation chirurgicale.

Les mécanismes à l'origine des douleurs du membre fantôme chez les amputés ne sont pas entièrement compris à ce jour. Il semble toutefois que le système nerveux central et périphérique y joue un rôle important, notamment par l'émergence de nouvelles connexions neuronales dans le cerveau et/ou de changements au niveau des neurones de la corne dorsale.

La demande WO2018/197307 (déposée par Algotherapeutix) et l'article de Rossignol, J. et al. (« High concentration of topical amitriptyline for treating chemotherapy-induced neuropathies ». Support Care Cancer 27, 3053-3059 (2019)), décrivent l'utilisation de compositions topiques sous forme de crèmes à concentration élevée en amitriptyline pour le traitement des douleurs neuropathiques périphériques post-chimiothérapie.

Diverses thérapies ont déjà été envisagées pour le traitement des douleurs du membre fantôme. Cependant, à l'heure actuelle, les traitements connus ne sont pas satisfaisants, comme le rapporte l'article de Nikolajsen, L et al. ("Phantom limb pain". British Journal of Anaesthesia, 2001, 87(1), pp.107-116).

En particulier, l'efficacité à court et à long terme de la toxine botulique A, des opioïdes, des antidépresseurs, des antagonistes des récepteurs NMDA, des anticonvulsivants, des calcitonines et des anesthésiques locaux, pour des résultats cliniquement pertinents notamment en termes de douleur, de qualité de vie, de satisfaction du traitement et des effets indésirables, reste incertaine.

Plus particulièrement, l'amitriptyline par voie orale avait été envisagé à une époque comme un possible traitement des douleurs du membre fantôme.

Toutefois, le traitement par l'amitriptyline par voie orale n'était pas satisfaisant et l'étude scientifique de Robinson, L. R. et al. (« Trial of Amitriptyline for Relief of Pain in Amputees: Results of a Randomized Controlled Study 1 ». Archives of Physical Medicine and Rehabilitation 85, no 1 (2004): 1 6) avait notamment conclue que les résultats obtenus ne sont pas en faveur d'une utilisation de l'amitriptyline pour le traitement des douleurs du membre fantôme, et qu'il est nécessaire de réaliser des études additionnelles pour développer et tester d'autres éventuels traitements.

Il existe donc un réel besoin de mettre au point des compositions pharmaceutiques qui soient efficaces dans le traitement des douleurs neuropathiques du membre fantôme.

Il est également d'intérêt que ces compositions pharmaceutiques induisent peu, voire pas, d'effets indésirables centraux.

Il a été découvert de façon surprenante que des compositions à base d'amitriptyline appliquées par voie topique permettait de traiter efficacement les douleurs neuropathiques du membre fantôme.

Cette découverte est d'autant plus surprenante que les douleurs neuropathiques du membre fantôme et les douleurs neuropathiques induites par chimiothérapie n'ont vraisemblablement pas la même origine.

En effet, les douleurs neuropathiques induites par chimiothérapie sont principalement dues à la toxicité chimique intrinsèque des molécules utilisées en chimiothérapie, qui va porter directement atteinte aux axones et qui peut engendrer des démyélinisations des nerfs.

Alors que les douleurs neuropathiques du membre fantôme semblent être la conséquence de la plasticité cérébrale et/ou de modifications dans les mécanismes neurophysiologiques à l'origine de la transmission de la douleur.

Les compositions sous forme de crème décrites dans la demande WO2018/197307 et dans l'article de Rossignol, J. et al*,* ne donnent toutefois pas une entière satisfaction en termes de stabilité dans le temps.

En effet, les crèmes sont les formes galéniques couramment utilisées pour l'administration de principes actifs par voie topique car celles-ci procurent généralement un meilleur passage transdermique et une bonne solubilisation de tous les actifs. Cependant, la stabilité physico-chimique de la composition selon la demande WO2018/197307 sous forme de crème, et plus précisément sous forme d'émulsion huile-dans-eau, n'est pas satisfaisante, notamment pour son utilisation en tant que médicament.

Le chlorhydrate d'amitriptyline est une molécule amphiphile qui est soluble dans l'eau sous forme de sel. Or il a été découvert de façon inattendue que la présence de ces électrolytes est déstabilisant pour les émulsions huile-dans-eau, a priori par masquage des charges de surface des globules d'huile ainsi que par rupture des équilibres d'interface huile/eau. Cette déstabilisation induit un déphasage de l'émulsion et à terme une séparation totale de l'huile et de l'eau. Dans le même temps, une réaction chimique se produit, se traduisant par un jaunissement de la couleur initialement blanche de l'émulsion. Ce déphasage et ce jaunissement sont problématiques pour une éventuelle mise sur le marché de la composition, notamment en tant que médicament.

Il existe donc un réel besoin de mettre au point des compositions pharmaceutiques qui soient efficaces dans le traitement des douleurs neuropathiques du membre fantôme, qui induisent peu, voire pas, d'effets indésirables centraux, et qui soient particulièrement stables dans le temps et à la température.

Tout particulièrement, il est d'intérêt que la composition pharmaceutique présente une bonne stabilité physico-chimique même à haute concentration en amitriptyline (i.e. même à une concentration d'au moins 10% en poids d'amitriptyline par rapport au poids total de la composition).

Il est également d'intérêt que la composition présente de bonnes qualités d'usage.

Or, il a été découvert de manière surprenante qu'une composition pharmaceutique topique sous forme de gel aqueux comprenant au moins de l'amitriptyline et/ou l'un de ses sels pharmaceutiquement acceptable, en une teneur totale en amitriptyline et/ou en l'un de ses sels pharmaceutiquement acceptable est comprise entre 10 et 30% en poids par rapport au poids total de la composition, permet de traiter efficacement les douleurs neuropathiques du membre fantôme, et présente une bonne stabilité physico-chimique dans le temps.

L'invention a donc pour objet une composition pharmaceutique sous forme de gel aqueux comprenant au moins de l'amitriptyline et/ou l'un de ses sels pharmaceutiquement acceptable, dont la teneur totale en amitriptyline et/ou en l'un de ses sels pharmaceutiquement acceptable est comprise entre 10 et 30% en poids par rapport au poids total de la composition, pour son utilisation par voie topique dans le traitement des douleurs neuropathiques du membre fantôme.

Il a été constaté que la composition selon l'invention est particulièrement efficace pour le traitement des douleurs neuropathiques du membre fantôme.

Tout particulièrement, la composition selon l'invention est grandement efficace pour le traitement des douleurs neuropathiques apparues à la suite de l'amputation ou de la désafférentation d'un membre (par exemple une main, un avant-bras ou une jambe).

Il a également été constaté que la composition pharmaceutique selon l'invention permet de faciliter la pénétration de l'amitriptyline à travers la peau avec un faible passage systémique, et ainsi d'obtenir une bonne efficacité thérapeutique.

La composition selon l'invention présente par ailleurs une biodisponibilité améliorée, de préférence à des concentrations en amitriptyline et/ou en l'un de ses sels pharmaceutiquement acceptables de 10 à 25% en poids et en particulier entre 10 et 20% en poids par rapport au poids total de la composition. En effet, à forte concentration l'amitriptyline a tendance à se réarranger, entrainant la formation d'agrégats susceptibles de limiter la biodisponibilité.

En outre, les compositions selon l'invention sont particulièrement stables dans le temps à température ambiante mais également à des températures de stockage plus élevées. Les compositions selon l'invention ont été soumises à des études de stabilités en conditions de température ambiante (25°C) pendant 24 mois minimum et accélérée (40°C) pendant 6 mois. Il résulte de ces études que les compositions selon l'invention n'ont pas changé d'aspect visuel ni physiquement (viscosité) ni chimiquement (pH, dosage du principe actif et des produits de dégradation).

Une composition selon l'invention a également été soumise à des dégradations forcées dans des conditions acidité forte, alcalinité forte, chaleur, lumière et condition oxydative. Les produits de dégradations observés sont restés dans des seuils acceptables dans la norme ICH Q3B (International Council for Harmonisation).

En outre, les compositions selon l'invention comprennent peu d'excipients, ce qui favorise une bonne tolérance locale de la composition (moins de risque d'allergie, moins de risque d'irritation). L'application topique de la composition selon l'invention présente peu, voire ne présente pas d'effets secondaires.

Les compositions selon l'invention présentent également de bonnes propriétés d'usage, à savoir les compositions sont translucides, inodores et agréables au toucher (toucher non gras).

De plus, les compositions selon l'invention s'administrent très facilement en flacon pompe. Ces flacons pompes sont notamment utiles pour assurer une bonne reproductibilité et une bonne précision de la dose administrée en principe actif.

En outre, la composition selon l'invention pourrait en plus de pallier aux douleurs, permettre de retrouver une peau plus saine et hydratée.

D'autres caractéristiques, aspects et avantages de l'invention apparaîtront encore plus clairement à la lecture de la description et des exemples qui suivent.

Dans la présente description, et à moins d'une indication contraire :
- l'expression "au moins un" est équivalente à l'expression "un ou plusieurs" et peut y être substituée ;
- l'expression "compris entre... et..." est équivalente à l'expression "allant de... à..." et peut y être substituée, et sous-entend que les bornes sont incluses ;
- l'expression « alcool gras » désigne un alcool comprenant de 9 à 40 atomes de carbone ;
- l'expression « acide gras » désigne un acide comprenant de 9 à 40 atomes de carbone ;
- l'expression « éther gras » désigne un éther comprenant de 9 à 40 atomes de carbone ;
- l'expression « ester gras » désigne un ester comprenant de 9 à 40 atomes de carbone ;
- l'expression « polyoxyalkyléné » correspond, au sens de l'invention, à un motif -(O-alkyle)ₙ-, où n est un nombre entier variant de 2 à 200, de préférence de 2 à 40, plus préférentiellement de 2 à 20, et où alkyle représente de préférence un éthyle ou un propyle ;
- l'expression « polyoxyéthyléné » correspond, au sens de l'invention, à un motif -(O-CH₂CH₂)ₙ₋, où n est un nombre entier variant de 2 à 200, de préférence de 2 à 40, plus préférentiellement de 2 à 20.

La composition pharmaceutique selon l'invention est utilisée dans le traitement par voie topique des douleurs du membre fantôme.

Avantageusement, la composition pharmaceutique selon l'invention est de préférence utilisée par voie cutanée. Autrement dit, la composition pharmaceutique est de préférence appliquée sur la peau.

Avantageusement, la composition pharmaceutique selon l'invention est de préférence utilisée dans le traitement par voie topique des douleurs neuropathiques apparues à la suite d'au moins une amputation et/ou d'au moins une désafférentation d'un membre (par exemple une main, un bras, un avant-bras ou une jambe).

En particulier, la composition pharmaceutique peut aussi bien être utilisée à titre préventif, par exemple chez les personnes ayant subi au moins une amputation et/ou au moins une désafférentation d'un membre et ne ressentant pas encore de douleurs neuropathiques, et/ou à titre curatif, par exemple chez les personnes ayant subi au moins une amputation et/ou au moins une désafférentation d'un membre et ressentant déjà des douleurs neuropathiques.

Plus particulièrement, la composition pharmaceutique selon l'invention peut être appliquée à titre préventif directement après le retrait des points de suture d'une amputation, soit de préférence 30 secondes à 12 heures après le retrait des points de suture, puis à nouveau une ou plusieurs fois ultérieurement.

### Amitriptyline

La composition selon la présente invention comprend au moins de l'amitriptyline et/ou l'un de ses sels pharmaceutiquement acceptable.

Selon l'invention, la teneur totale en amitriptyline et/ou en l'un de ses sels pharmaceutiquement acceptable est comprise entre 10 et 30% en poids par rapport au poids total de la composition.

L'amitriptyline est de formule (I) suivante :

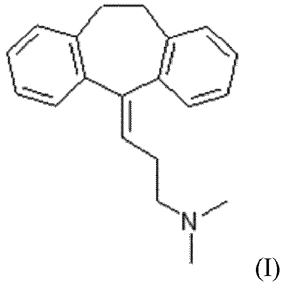

Dans le cadre de la présente invention, on entend par « *sels d'amitriptyline pharmaceutiquement acceptable* », les sels compatibles avec une composition pharmaceutique c'est-à-dire destinée à être administrée à des humains. En particulier, on entend par sel d'amitriptyline pharmaceutiquement acceptable les hydrates, les solvates, les sels d'acides tel que les chlorhydrates et les clathrates de l'amitriptyline.

Comme sel tout particulièrement préféré de l'amitriptyline, on utilisera le chlorhydrate d'amitriptyline.

De préférence, la teneur totale en amitriptyline et/ou en l'un de ses sels pharmaceutiquement acceptable est comprise entre 10 et 25% en poids, plus préférentiellement entre 10 et 20% en poids, plus préférentiellement encore entre 10 et 15% en poids, par rapport au poids total de la composition.

Plus préférentiellement, la teneur totale en chlorhydrate d'amitriptyline est comprise entre 10 et 25% en poids, plus préférentiellement encore entre 10 et 20% en poids, encore mieux entre 10 et 15% en poids, par rapport au poids total de la composition.

Tout particulièrement, il a été observé de façon surprenante que lorsque la teneur totale en amitriptyline, et/ou en l'un de ses sels pharmaceutiquement acceptables tel que le chlorhydrate d'amitriptyline, est comprise entre 10 et 25% en poids, plus préférentiellement entre 10 et 20% en poids, par rapport au poids total de la composition selon l'invention, alors la biodisponibilité en amitriptyline de la composition selon l'invention est significativement améliorée.

En effet, il a été observé qu'à forte concentration l'amitriptyline a tendance à se réarranger, entrainant la formation d'agrégats susceptibles de limiter la biodisponibilité.

De préférence, la composition selon l'invention contient l'amitriptyline et/ou l'un de ses sels pharmaceutiquement acceptable dans les proportions précitées, comme seul agent traitant la douleur.

La composition pharmaceutique selon l'invention est sous forme de gel aqueux.

Selon le Clinical Data Interchange Standards Consortium (CDISC), un gel pharmaceutique est une forme galénique semi-solide contenant un agent gélifiant pour donner de la rigidité à une solution ou à une dispersion colloïdale. Un gel peut contenir des particules en suspension.

Au sens de l'invention, il est entendu que les compositions sous forme de gel aqueux selon l'invention comprennent les compositions aqueuses visqueuses dont la viscosité est comprise entre 400 et 2500 mPa.s (à une température de 20°C et à pression atmosphérique).

De préférence, la viscosité des compositions sous forme de gel aqueux selon l'invention, à une température de 20°C et à pression atmosphérique, est comprise entre 400 et 2500 mPa.s ; plus préférentiellement entre 600 et 2000 mPa.s ; et plus préférentiellement encore entre 800 et 1500 mPa.s.

A titre d'exemple, la viscosité des compositions sous forme de gel aqueux selon l'invention est déterminée au moyen d'un viscosimètre Brookfield LV, en utilisant le mobile numéro 63, tournant à la vitesse de 50 tpm (tours par minute), à une température 20,0°C +/- 2,0°C) dans un récipient de 30 mL, de 40 mm de hauteur et 35 mm de diamètre. Lorsque le viscosimètre est étalonné, le mobile est immergé dans le gel jusqu'à ce qu'un centimètre du fond de la bouteille. La viscosité est prise lorsque la mesure est stable.

La composition selon l'invention n'est pas sous forme d'émulsion, telle que par exemple une émulsion huile-dans-eau ou une émulsion eau-dans-huile. Autrement dit, la composition selon l'invention ne comprend pas de phase huileuse.

Avantageusement, la composition selon l'invention est exempte de corps gras.

Au sens de l'invention, on entend par « corps gras », un composé organique insoluble dans l'eau à 25°C et à pression atmosphérique (760 mm de Hg, soit 1,013.10⁵ Pa), c'est-à-dire de solubilité dans l'eau inférieure à 5% et de préférence inférieure à 1%, encore plus préférentiellement inférieure à 0,1%. A titre d'exemples de corps gras, on peut citer les cires, les hydrocarbures, les alcools gras comprenant de 9 à 40 atomes de carbone, les acides gras comprenant de 9 à 40 atomes de carbone, les esters gras comprenant de 9 à 40 atomes de carbone, les éthers gras comprenant de préférence de 9 à 40 atomes de carbone, les silicones et leurs mélanges.

La composition selon l'invention comprend de l'eau.

De préférence, la teneur totale en eau est supérieure ou égale à 65% en poids, plus préférentiellement comprise entre 65 et 90% en poids ; plus préférentiellement encore entre 70 et 90% en poids, encore mieux entre 75 et 85% en poids, par rapport au poids total de la composition selon l'invention.

De préférence, la composition selon l'invention comprend au moins un polymère cellulosique.

Par polymère « cellulosique », on entend selon l'invention tout composé polysaccharidique, substitué ou non, possédant dans sa structure des enchaînements de résidus glucose unis par des liaisons β-1,4 ; outre les celluloses non substituées, les dérivés de celluloses peuvent être anioniques, cationiques, amphotères ou non-ioniques.

Ainsi, les polymères cellulosiques utilisables selon l'invention peuvent être choisis parmi les celluloses non substituées y compris sous une forme microcristalline et les celluloses substituées.

Plus préférentiellement, les polymères cellulosiques utilisables ne comportent pas de chaîne grasse latérale en C₁₀-C₃₀ dans leur structure.

De préférence, le ou les polymères cellulosiques utilisables présentent un poids moléculaire moyen compris entre 5 000 et 1 500 000, plus préférentiellement entre 50 000 et 800 000, plus préférentiellement encore entre 400 000 et 800 000.

Parmi les polymères cellulosiques utilisables selon l'invention, on peut distinguer les éthers de celluloses, les esters de celluloses et les esters éthers de celluloses.

Parmi les esters de celluloses, on trouve les esters inorganiques de cellulose (nitrates, sulfates ou phosphates de cellulose...), les esters organiques de cellulose (monoacétates, triacétates, amidopropionates, acétatebutyrates, acétatepropionates ou acétatetrimellitates de cellulose....) et les esters mixtes organique/inorganique de cellulose tels que les acétatebutyratesulfates et les acétatepropionatesulfates de cellulose. Parmi les esters éthers de cellulose, on peut citer les phtalates d'hydroxypropylméthylcellulose et les sulfates d'éthylcellulose.

Parmi les éthers de cellulose non-ioniques, on peut citer les (Ci-C₄)alkylcelluloses telles que les méthylcelluloses et les éthylcelluloses (par exemple Ethocel standard 100 Premium de DOW CHEMICAL) ; les (poly)hydroxy(C₁-C₄)alkylcelluloses telles que les hydroxyméthylcelluloses, les hydroxyéthylcelluloses (par exemple Natrosol 250 HHR proposé par AQUALON) et les hydroxypropylcelluloses (par exemple Klucel EF d'AQUALON) ; les celluloses mixtes (poly)hydroxy(C₁-C₄)alkyl-(C₁-C₄)alkylcelluloses telles que les hydroxypropyl-méthylcelluloses (par exemple Methocel E4M de DOW CHEMICAL), les hydroxyéthyl-méthylcelluloses, les hydroxyéthyl-éthylcelluloses (par exemple Bermocoll E 481 FQ d'AKZO NOBEL) et les hydroxybutyl-méthylcelluloses.

Parmi les éthers de cellulose anioniques, on peut citer les (poly)carboxy(C₁-C₄)alkylcelluloses et leurs sels. A titre d'exemple, on peut citer les carboxyméthylcelluloses, les carboxyméthylméthylcelluloses (par exemple Blanose 7M de la société AQUALON) et les carboxyméthylhydroxyéthylcelluloses et leurs sels de sodium.

Parmi les éthers de cellulose cationiques, on peut citer les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les (poly)hydroxy(C₁-C₄)alkyl celluloses, comme les hydroxyméthyl-, hydroxyéthyl- ou hydroxypropyl celluloses greffées notamment avec un sel de méthacryloyléthyl-triméthylammonium, de méthacrylmidopropyl-triméthylammonium, de diméthyl-diallylammonium. Les produits commercialisés répondant à cette définition sont plus particulièrement les produits vendus sous la dénomination « Celquat^{®} L 200 » et « Celquat^{®} H 100 » par la Société National Starch.

De préférence, le ou les polymères cellulosiques sont choisis parmi les polymères cellulosiques ne comportent pas de chaîne grasse latérale en C₁₀-C₃₀ dans leur structure ; plus préférentiellement parmi les éthers de cellulose ; plus préférentiellement encore parmi les éthers de cellulose non-ioniques ; encore mieux parmi (a) les (C₁-C₄)alkylcelluloses telles que les méthylcelluloses et les éthylcelluloses, (b) les (poly)hydroxy(C₁-C₄)alkylcelluloses telles que les hydroxyméthylcelluloses, les hydroxyéthylcelluloses et les hydroxypropylcelluloses, (c) les celluloses mixtes (poly)hydroxy(C₁-C₄)alkyl-(C₁-C₄)alkylcelluloses telles que les hydroxypropyl-méthylcelluloses, les hydroxypropyl-éthylcelluloses, les hydroxyéthyl-méthylcelluloses, les hydroxyéthyl-éthylcelluloses et les hydroxybutyl-méthylcelluloses, et (d) leurs mélanges.

Plus préférentiellement, la composition selon l'invention comprend au moins une (poly)hydroxy(C₁-C₄)alkylcellulose telles que les hydroxyméthylcelluloses, les hydroxyéthylcelluloses et les hydroxypropylcelluloses ; encore mieux au moins l'hydroxyéthylcellulose.

De préférence, lorsque la composition selon l'invention comprend au moins un polymère cellulosique, la teneur totale en polymère(s) cellulosique(s) est comprise entre 0,1 et 10% en poids, plus préférentiellement entre 0,5 et 5% en poids, plus préférentiellement encore entre 1 et 2,5% en poids, par rapport au poids total de la composition selon l'invention.

De préférence, lorsque la composition selon l'invention comprend au moins un polymère cellulosique, la teneur totale en (poly)hydroxy(C₁-C₄)alkylcellulose(s) est comprise entre 0,1 et 10% en poids, plus préférentiellement entre 0,5 et 5% en poids, plus préférentiellement encore entre 1 et 2,5% en poids, par rapport au poids total de la composition selon l'invention.

De préférence, lorsque la composition selon l'invention comprend au moins un polymère cellulosique, la teneur totale en hydroxyéthylcellulose est comprise entre 0,1 et 10% en poids, plus préférentiellement entre 0,5 et 5% en poids, plus préférentiellement encore entre 1 et 2,5% en poids, par rapport au poids total de la composition selon l'invention.

De préférence, la composition selon l'invention comprend au moins un polyol en C₂-C₈.

Par « polyol en C₂-C₈ » au sens de la présente invention, on entend un composé organique constitué d'une chaîne hydrocarbonée en C₂-C₈, éventuellement interrompue par un ou plusieurs atomes d'oxygène, et porteuse d'au moins deux groupements hydroxyles libres (-OH) portés par des atomes de carbone différents, ce composé pouvant être cyclique ou acyclique, linéaire ou ramifié, saturé ou insaturé, et à l'état liquide à température ambiante (25°C) et à pression atmosphérique (soit 1,013.10⁵ Pa).

De préférence, le ou les polyols en C₂-C₈ utilisables sont acycliques et non-aromatiques.

Les polyols en C₂-C₈ utilisables comprennent dans leur structure de 2 à 8 atomes de carbone, de préférence de 2 à 6 atomes de carbone, plus préférentiellement de 2 à 5 atomes de carbone.

Plus particulièrement, le ou les polyols utilisables comprennent de 2 à 10 groupements hydroxy, plus préférentiellement de 2 à 5 groupements hydroxy, plus préférentiellement encore de 2 à 3 groupements hydroxy.

De manière préférée, le ou lesdits polyols en C₂-C₈ utilisables sont choisis parmi les polyols en C₃-C₆, l'éthylène glycol, et leurs mélanges.

Plus préférentiellement, le ou lesdits polyols en C₂-C₈ utilisables selon l'invention sont choisis parmi le propylène glycol, le 1,3-propanediol, le 1,3-butylène glycol, le pentane-1,2-diol, le dipropylène glycol, l'hexylène glycol, le pentylène glycol, le glycérol, l'éthylène glycol, et un mélange de ces composés ; plus préférentiellement la composition comprend au moins le propylène glycol.

De préférence, lorsque la composition selon l'invention comprend au moins un polyol en C₂-C₈, la teneur totale en polyol(s) en C₂-C₈ est comprise entre 0,1 et 15% en poids, plus préférentiellement entre 0,5 et 10% en poids, plus préférentiellement encore entre 1 et 6% en poids, et encore mieux entre 3 et 6% en poids, par rapport au poids total de la composition selon l'invention.

De préférence, lorsque la composition selon l'invention comprend au moins un polyol en C₂-C₈, la teneur totale en propylène glycol est comprise entre 0,1 et 15% en poids, plus préférentiellement entre 0,5 et 10% en poids, plus préférentiellement encore entre 1 et 6% en poids, et encore mieux entre 3 et 6% en poids, par rapport au poids total de la composition selon l'invention.

De préférence, lorsque la composition selon l'invention comprend au moins un polymère cellulosique et au moins un polyol en C₂-C₈, le rapport pondéral de la teneur totale en polyol(s) en C₂-C₈ d'une part sur la teneur totale polymère(s) cellulosique(s) d'autre part, va de 0,01 à 150, plus préférentiellement de 0,1 à 20, plus préférentiellement encore de 0,4 à 6, mieux encore de 1 à 6, voire de 1,2 à 6.

Avantageusement, lorsque la composition selon l'invention comprend au moins un polymère cellulosique et au moins un polyol en C₂-C₈, la teneur totale en poids en polymère(s) cellulosique(s) est strictement inférieure à la teneur totale en poids en polyol(s) en C₂-C₈.

De préférence, la composition selon l'invention est exempte de tensioactif.

Selon un mode de réalisation particulier de l'invention, la composition peut éventuellement comprendre en outre au moins un tensioactif.

Les tensioactifs utilisables selon l'invention peuvent être choisis parmi les tensioactifs anioniques, les tensioactifs cationiques, les tensioactifs amphotères et/ou zwittérioniques, les tensioactifs non ioniques, et leurs mélanges.

Plus préférentiellement, le ou les tensioactifs utilisables selon l'invention sont choisis parmi les tensioactifs non ioniques.

Les tensioactifs non ioniques utilisables peuvent être choisis parmi les alkyl polyglucosides (APG), les esters de glycérol oxyalkylénés, les esters d'acides gras et de sorbitan éventuellement oxyalkylénés, les esters d'acides gras polyoxyalkylénés (en particulier polyoxyéthylénés et/ou polyoxypropylénés) éventuellement en association avec un ester d'acide gras et de glycérol comme le mélange PEG-100 Stearate / Glyceryl Stearate commercialisé par exemple par la société ICI sous la dénomination Arlacel 165, les esters de sucre oxyalkylénés, et leurs mélanges.

Comme alkylpolyglucosides, on peut citer ceux contenant un groupe alkyle comportant de 6 à 30 atomes de carbone et de préférence de 8 à 16 atomes de carbone, et contenant un groupe glucoside comprenant de préférence 1,2 à 3 unités de glucoside. Les alkylpolyglucosides peuvent être choisis par exemple parmi le decylglucoside (Alkyl-C₉/C₁₁-polyglucoside (1.4)) comme le produit commercialisé sous la dénomination Mydol 10^{®} par la société Kao Chemicals ou le produit commercialisé sous la dénomination Plantacare 2000 UP^{®} par la société Cognis ; le caprylyl/capryl glucoside comme le produit commercialisé sous la dénomination Plantacare KE 3711^{®} par la société Cognis ; le laurylglucoside comme le produit commercialisé sous la dénomination Plantacare 1200 UP^{®} par la société Cognis ; le cocoglucoside comme le produit commercialisé sous la dénomination Plantacare 818 UP^{®} par la société Cognis ; le caprylylglucoside comme le produit commercialisé sous la dénomination Plantacare 810 UP^{®} par la société Cognis ; et leurs mélanges.

Les esters de glycérol oxyalkylénés sont notamment les dérivés polyoxyéthylénés des esters de glycéryle et d'acide gras et de leurs dérivés hydrogénés. Ces esters de glycérol oxyalkylénés peuvent être choisis par exemple parmi les esters de glycéryle et d'acides gras hydrogénés et oxyéthylénés tel que le PEG-200 hydrogenated glyceryl palmate commercialisé sous la dénomination Rewoderm LI-S 80 par la société Goldschmidt ; les cocoates de glycéryle oxyéthylénés comme le PEG-7 glyceryl cocoate commercialisé sous la dénomination Tegosoft GC par la société Goldschmidt, et le PEG-30 glyceryl cocoate commercialisé sous la dénomination Rewoderm LI-63 par la société Goldschmidt ; les stéarates de glycéryle oxyéthylénés ; et leurs mélanges.

Les esters de sucres oxyalkylénés sont notamment les éthers de polyéthylène glycol des esters d'acide gras et de sucre. Ces esters de sucre oxyalkylénés peuvent être choisis par exemple parmi les esters de glucose oxyéthylénés tels que le PEG-120 methyl glucose dioleate commercialisé sous la dénomination Glucamate DOE 120 par la société Amerchol.

De préférence, le nombre de moles d'oxyde d'alkylène des tensioactifs non ioniques utilisables selon l'invention varie de 2 à 400 ; plus préférentiellement de 4 à 250.

Selon une variante de l'invention, la composition comprend au moins un tensioactif non ionique ; plus préférentiellement un tensioactif non ionique choisi parmi les esters de glycérol polyoxyalkylénés ; plus préférentiellement encore au moins un tensioactif non ionique choisi parmi les esters de glycéryle et d'acides gras hydrogénés et polyoxyéthylénés tel que le PEG-200 hydrogenated glyceryl palmate, les cocoates de glycéryle polyoxyéthylénés tels que le PEG-7 glyceryl cocoate et le PEG-30 glyceryl cocoate, les stéarates de glycéryle polyoxyéthylénés, et leurs mélanges. Plus préférentiellement encore selon cette variante, la composition comprend au moins un cocoate de glycéryle polyoxyéthyléné.

De préférence, lorsque la composition selon l'invention comprend au moins un tensioactif, la teneur totale en tensioactif(s) est comprise entre 0,1 et 10% en poids, plus préférentiellement entre 0,5 et 5% en poids, plus préférentiellement encore entre 1 et 4% en poids, par rapport au poids total de la composition selon l'invention.

De préférence, lorsque la composition selon l'invention comprend au moins un tensioactif, la teneur totale en tensioactif(s) non ionique(s) est comprise entre 0,1 et 10% en poids, plus préférentiellement entre 0,5 et 5% en poids, plus préférentiellement encore entre 1 et 4% en poids, par rapport au poids total de la composition selon l'invention.

De préférence, lorsque la composition selon l'invention comprend au moins un tensioactif, la teneur totale en ester(s) de glycérol (poly)oxyalkyléné(s) est comprise entre 0,1 et 10% en poids, plus préférentiellement entre 0,5 et 5% en poids, plus préférentiellement encore entre 1 et 4% en poids, par rapport au poids total de la composition selon l'invention.

De préférence, la composition selon l'invention est exempte d'agent anti-oxydant.

Selon une variante de l'invention, la composition comprend en outre au moins un agent anti-oxydant ; plus préférentiellement choisi parmi le tocophérol et ses esters, tels que l'acétate de tocophérol, le gallate de propyle, l'hydroxytoluène butylée (BHT), l'hydroxyanisole butylé (BHA), et leurs mélanges.

De préférence, la composition selon l'invention est exempte d'agent séquestrant.

Selon une variante de l'invention, la composition comprend en outre au moins un agent séquestrant ; plus préférentiellement choisi parmi (a) l'acide éthylènediamine tétracétique (EDTA) et ses sels tel que le sel disodique d'éthylène diaminetétracétique (Disodium EDTA), (b) les dérivés phosphoniques et leurs sels tels que l'acide hexaméthylène diaminetétra(méthylène phosphonique), l'acide éthylènediamine tétra(méthylène phosphonique), l'acide 1-hydroxyéthylidène-1,1-diphosphonique, l'acide aminotri(méthylènephosphonique), l'acide diéthylène-triamine penta(méthylène phosphonique), (c) les polymères polyaminés tels que les polyalkylène polyamines et leurs dérivés, en particulier la polyéthylèneimine, (d) les dendrimères à activité chélatante, (e) les protéines comme la spermine, la spermidine, la transférine, la ferritine, (f) les acides carboxyliques comme l'acide phytique, l'acide citrique, l'acide malique, l'acide nitrilo-acétique, l'acide fumarique, l'acide tartrique, l'acide succinique, l'acide oxalique, (g) la desferrioxamine mesylate, et leurs mélanges.

La définition d'« agent séquestrant » (également appelé « agent chélatant ») est bien connu de l'homme du métier et fait référence à un composé ou un mélange de composés capable(s) de former un chélate avec un ion métallique. Un chélate est un complexe inorganique dans lequel un composé (l'agent séquestrant ou chélatant) est coordiné à un ion métallique, c'est-à-dire qu'il forme une ou plusieurs liaisons avec l'ion métallique (formation d'un cycle incluant l'ion métallique).

Un agent séquestrant (ou chélatant) comprend généralement au moins deux atomes donneurs d'électrons qui permettent la formation de liaisons avec l'ion métallique.

Selon une autre variante de l'invention, la composition comprend au moins un agent séquestrant et au moins un agent anti-oxydant.

Selon une autre variante de l'invention, la composition est exempte de corps gras, d'agent séquestrant et/ou d'agent anti-oxydant.

De préférence, le pH de la composition selon l'invention est compris entre 3 et 8, plus préférentiellement entre 4 et 7, et encore mieux entre 5 et 6.

Le pH de ces compositions peut être ajusté à la valeur désirée au moyen d'agents alcalinisants ou d'agents acidifiants habituellement utilisés. Parmi les agents alcalinisants, on peut citer, à titre d'exemples, l'ammoniaque, les alcanolamines, les hydroxydes minéraux ou organiques. Parmi les agents acidifiants, on peut citer, à titre d'exemples, les acides minéraux ou organiques comme l'acide chlorhydrique, l'acide orthophosphorique, les acides carboxyliques comme par exemple l'acide acétique, l'acide tartrique, l'acide citrique, l'acide lactique, les acides sulfoniques.

La composition selon l'invention peut contenir en outre des additifs ou excipients habituellement utilisés en pharmaceutique, comme un ou plusieurs parfums, tampons, des colorants, des antibactériens et/ou antifongiques.

Comme antibactérien, les parabènes sont de préférence utilisés, et plus préférentiellement le méthyl-parabène.

Ces additifs ou excipients peuvent être présents dans la composition selon l'invention en une quantité allant de 0 à 20 % en poids par rapport au poids total de la composition.

L'homme de métier veillera à choisir ces éventuels additifs ou excipients et leurs quantités de manière à ce qu'ils ne nuisent pas aux propriétés des compositions de la présente invention.

De façon particulièrement préférée, la composition pharmaceutique sous forme de gel aqueux pour une application topique selon l'invention est la composition CP1 suivante, comprenant :
- de 10 à 30% en poids, de préférence de 10 à 25 % en poids, plus préférentiellement de 10 à 20% en poids, plus préférentiellement encore de 10 à 15% en poids, d'amitriptyline ou de l'un de ses sels pharmaceutiquement acceptable, par rapport au poids total de la composition,
- de 0,1 à 10% en poids, de préférence de 0,5 à 5% en poids, plus préférentiellement encore de 1 à 2,5% en poids, d'au moins un polymère cellulosique tel que décrit précédemment, par rapport au poids total de la composition,
- de 0,1 à 15% en poids, de préférence de 0,5 à 10% en poids, plus préférentiellement encore de 1 à 6% en poids, et encore mieux de 3 à 6% en poids, d'au moins un polyol en C₂-C₈ tel que décrit précédemment, par rapport au poids total de la composition,
- optionnellement de 0,1 à 10% en poids, plus préférentiellement de 0,5 à 5% en poids, plus préférentiellement encore de 1 à 4% en poids, d'au moins un tensioactif non ionique tel que décrit précédemment, par rapport au poids total de la composition,
- optionnellement de 0 à 3% en poids d'au moins un agent séquestrant et/ou au moins un agent anti-oxydant tels que décrits précédemment,
- optionnellement de 0,01 à 0,5% en poids d'au moins un agent antibactérien, de préférence le méthyl-parabène,
- optionnellement de 0 à 1% en poids d'un ou plusieurs ajusteurs de pH tel que décrit précédemment, de façon à maintenir le pH entre 3 et 8, de préférence entre 4 et 7, plus préférentiellement entre 5 et 6.
- de l'eau, en une teneur totale supérieure ou égale à 65% en poids, de préférence comprise entre 65 et 90% en poids ; plus préférentiellement entre 70 et 90% en poids, plus préférentiellement encore entre 75 et 85% en poids, par rapport au poids total de la composition.

Selon une variante de cette composition CP1, la composition CPlest exempte de corps gras, de tensioactif, d'agent séquestrant et/ou d'agent anti-oxydant.

De façon toute particulièrement préférée, la composition pharmaceutique sous forme de gel aqueux pour une application topique selon l'invention est la composition CP2 suivante, comprenant :
- de 10 à 30% en poids, de préférence de 10 à 25 % en poids, plus préférentiellement de 10 à 20% en poids, plus préférentiellement encore de 10 à 15% en poids, d'amitriptyline ou de l'un de ses sels pharmaceutiquement acceptable, par rapport au poids total de la composition,
- de 0,1 à 10% en poids, de préférence de 0,5 à 5% en poids, plus préférentiellement encore de 1 à 2,5% en poids, d'au moins un éther de cellulose non-ionique, de préférence de poids moléculaire moyen compris entre 50 000 et 800 000, tel que décrit précédemment, par rapport au poids total de la composition,
- de 0,1 à 15% en poids, de préférence de 0,5 à 10% en poids, plus préférentiellement encore de 1 à 6% en poids, et encore mieux de 3 à 6% en poids, d'au moins un polyol en C₂-C₈ choisi parmi le propylène glycol, le 1,3-propanediol, le 1,3-butylène glycol, le pentane-1,2-diol, le dipropylène glycol, l'hexylène glycol, le pentylène glycol, le glycérol, l'éthylène glycol, et un mélange de ces composés, par rapport au poids total de la composition,
- optionnellement de 0,1 à 10% en poids, plus préférentiellement de 0,5 à 5% en poids, plus préférentiellement encore de 1 à 4% en poids, d'au moins un ester de glycérol oxyalkyléné tel que décrit précédemment, par rapport au poids total de la composition,
- optionnellement de 0 à 3% en poids au moins un agent séquestrant et/ou au moins un agent anti-oxydant tels que décrits précédemment,
- optionnellement de 0,01 à 0,5% en poids d'au moins un agent antibactérien, de préférence le méthyl-parabène,
- optionnellement de 0 à 1% en poids d'un ou plusieurs ajusteurs de pH tel que décrit précédemment, de façon à maintenir le pH entre 3 et 8, de préférence entre 4 et 7, plus préférentiellement entre 5 et 6.
- de l'eau, en une teneur totale supérieure ou égale à 65% en poids, de préférence comprise entre 65 et 90% en poids ; plus préférentiellement entre 70 et 90% en poids, plus préférentiellement encore entre 75 et 85% en poids, par rapport au poids total de la composition.

Selon une variante de cette composition CP2, la composition CP2est exempte de corps gras, de tensioactif, d'agent séquestrant et/ou d'agent anti-oxydant.

Les compositions CP1 et CP2 sont particulièrement efficaces dans le traitement des douleurs neuropathiques du membre fantôme.

L'invention porte aussi sur une méthode de traitement par voie topique, de préférence par voie cutanée, des douleurs neuropathiques, notamment périphériques, du membre fantôme, comprenant une ou plusieurs applications topiques de la composition selon l'invention telle que décrite précédemment.

En particulier, la composition pharmaceutique peut aussi bien être appliquée à titre préventif, par exemple chez les personnes ayant subi au moins une amputation et/ou au moins une désafférentation d'un membre et ne ressentant pas encore de douleurs neuropathiques, et/ou à titre curatif, par exemple chez les personnes ayant subi au moins une amputation et/ou au moins une désafférentation d'un membre et ressentant déjà des douleurs neuropathiques.

Plus particulièrement, la composition pharmaceutique selon l'invention peut être appliquée à titre préventif directement après le retrait des points de suture d'une amputation, soit de préférence 30 secondes à 12 heures après le retrait des points de suture, puis à nouveau une ou plusieurs fois ultérieurement.

Les exemples suivants illustrent les compositions selon l'invention et les avantages de ces compositions. Ils ne représentent en rien une limitation de la présente invention.

### Exemples :

### Exemple 1 :

Etude *ex vivo* comparée de l'absorption percutanée de l'amitriptyline dans une formulation A sous forme de gel aqueux (invention) et dans une formulation B sous forme de crème (comparatif).

Le gel aqueux (composition A) suivant a été préparé à partir des ingrédients indiqués dans le tableau ci-après dont les quantités sont exprimées en % en poids.

**Tableau 1**

| **COMPOSITION A (Invention)** | **Quantité** |
|---|---|
| Chlorhydrate d'amitriptyline | 10 |
| Hydroxyéthylcellulose | 1 |
| Propylène glycol | 5 |
| PEG-7 Cocoate de glycéryle | 2 |
| Sel disodique d'éthylène diaminetétracétique | 0,1 |
| Gallate de propyle | 0,05 |
| Agent de pH | Qsp pH 5,5 ± 0,5 |
| Eau | Qsp 100 |

La composition B (crème) comprend 10% en poids de chlorhydrate d'amitriptyline et 90% en poids de crème Excipial Hydrocrème^{®}, commercialisée par la société Galderma, par rapport au poids total de la composition B.

Chacune des compositions A et B a été appliquées sur des échantillons distincts de peau humaine. Pour chaque composition, l'expérience a été répétée 3 fois avec 3 échantillons de peau de 3 donneurs différents, soit 9 échantillons.

Les échantillons de peau sont montés dans une cellule de Frantz et sont portées à une température de surface de 32°C±1°C.

La composition A ou B est étendue de façon homogène à l'aide d'une spatule sur chaque échantillon de peau à raison de 10 mg par cellule, correspondant à 5mg /cm² de peau.

On rince les échantillons de peau 16 heures après application.

Chaque échantillon de peau a été placé avec une pince à épiler sur un papier absorbant (derme vers le bas).

On enlève le stratum corneum en utilisant des bandes adhésives.

Après élimination du stratum corneum l'échantillon est perforé. L'épiderme est ensuite séparé du derme. Chacun d'eux est placé dans des fioles séparées.

On a procédé ensuite à l'extraction des différents échantillons.

Ce profil de pénétration a démontré son efficacité en clinique lors de l'étude décrite par l'article de Rossignol et al (« High concentration of topical amitriptyline for treating chemotherapy-induced neuropathies ». Support Care Cancer 27, 3053-3059 (2019)).

Les résultats de ces extractions sont regroupés dans le tableau ci-dessous.

**Tableau 2**

| | **Composition A (Invention)** | **Composition B (Comparatif)** |
|---|---|---|
| Concentration d'amitriptyline restant à la surface de la peau - stratum corneum *(µg)* | 2,4 ± 1,5 | 3,3 ± 1,6 |
| Concentration d'amitriptyline dans l'épiderme *(µg)* | 3,6 ± 2,6 | 4,1 ± 2,5 |
| Concentration d'amitriptyline dans le derme *(µg)* | 5,2 ± 2,5 | 4,4 ± 2,2 |
| Concentration d'amitriptyline dans le fluide récepteur (circulation sanguine) | 0,15 ± 0,08 | 0,13 ± 0,13 |
| Biodisponibilité *(µg*/*cm² de peau)* | 9,0 ±4,8 | 8,7 ± 4,0 |

Il a également été observé que le passage systémique de l'amitriptyline était inférieur à 0,1% de la dose administrée. Il en résulte que le passage systémique de l'amitriptyline est négligeable.

On remarque que le gel aqueux A selon l'invention présente un profil de pénétration de l'amitriptyline dans la peau satisfaisant et similaire au profil de pénétration de l'amitriptyline dans la peau obtenu avec la crème comparative B.

On note également que la biodisponibilité obtenue de la composition A et celle obtenue de la composition B sont similaires.

Il a également été observé que la composition A est particulièrement efficace dans le traitement des douleurs neuropathiques du membre fantôme.

### Exemple 2 :

Une autre composition pharmaceutique sous forme de gel aqueux selon l'invention (composition A') a été préparée à partir des ingrédients indiqués dans le tableau ci-après dont les quantités sont exprimées en % en poids.

**Tableau 3**

| **COMPOSITION A' (Invention)** | **Quantité** |
|---|---|
| Chlorhydrate d'amitriptyline | 15 |
| Hydroxyéthylcellulose | 1 |
| Propylène glycol | 5 |
| Methyl paraben | 0,1 |
| Agent de pH | Qsp pH 5,5 ± 0,5 |
| Eau | Qsp 100 |

Il a été observé que le gel aqueux A' selon l'invention présente un profil de pénétration de l'amitriptyline dans la peau et une biodisponibilité en amitriptyline satisfaisants.

Il a également été observé que la composition A' est particulièrement efficace dans le traitement des douleurs neuropathiques du membre fantôme.

### Exemple 3 :

Etude de la stabilité d'une formulation C sous forme de gel aqueux (invention) et de la formulation B sous forme de crème (comparatif).

Le gel aqueux (composition C) suivant a été préparé à partir des ingrédients indiqués dans le tableau ci-après dont les quantités sont exprimées en % en poids.

**Tableau 4**

| **COMPOSITION C (Invention)** | **Quantité** |
|---|---|
| Chlorhydrate d'amitriptyline | 10 |
| Hydroxyéthylcellulose | 1 |
| Propylène glycol | 5 |
| PEG-7 Cocoate de glycéryle | 2 |
| Agent de pH (NaOH, solution 1N) | Qsp pH 5,5 ± 0,5 |
| Eau | Qsp 100 |

La composition B (crème) comprend 10% en poids de chlorhydrate d'amitriptyline et 90% en poids de crème Excipial Hydrocrème^{®}, commercialisée par la société Galderma, par rapport au poids total de la composition B.

Chacune des compositions B et C ont été placées dans une étuve à une température de 40°C.

La stabilité des compositions a ensuite été évaluée visuellement dans le temps (à To, au moment de l'entrée en étuve ; à T₂₄ₕ, 24 heures après l'entrée en étuve ; et à T₃ₘₒᵢₛ, 3 mois après l'entrée en étuve).

Les résultats sont regroupés dans le tableau ci-dessous.

**Tableau 5**

| | **Aspect** | | |
|---|---|---|---|
| **Compositions** | à T₀ | à T24h | à T3mois |
| Composition B (Comparatif) | Emulsion blanche opaque huile-dans-eau | Déphasage observé. La phase huileuse supérieure est blanche et opaque. La phase aqueuse inférieure est transparente | ND |
| Composition C (Invention) | Gel translucide sans couleur | Gel translucide | Gel translucide |

Aucune synérèse n'a été observé pour la composition C sous forme de gel aqueux selon l'invention après 3 mois à 40°C.

On remarque également un déphasage de la composition comparative B sous forme d'émulsion huile-dans-eau au bout de seulement 24 heures à 40°C.

De plus, une étude de stabilité complète a été effectué sur la composition C selon l'invention pendant 6 mois à 40°C.

Les résultats sont regroupés dans les tableaux 6 et 7 ci-après.

**Tableau 6**

| **TESTS** | **SPECIFI-CATIONS** | **RESULTATS** | | | |
|---|---|---|---|---|---|
| | | **T0** | **T1 mois** | **T3 mois** | **T6 mois** |
| Aspect visuel | Gel translucide sans couleur | Gel translucide sans couleur | Gel translucide sans couleur | Gel translucide sans couleur | Gel translucide sans couleur |
| pH | 4.5 à 6.0 | 5.5 | 5.6 | 5.5 | 5.3 |
| Viscosité | 450 à 1500 mPa.s | 1039 mPa.s | 883 mPa.s | 765 mPa.s | 756 mPa.s |
| Dosage de amitriptyline HCl | 95,0 mg/g à 105,0 mg/g | 102,7 mg/g | 99,9 mg/g | 99,6 mg/g | 100,6 mg/g |

**Tableau 7**

| **Produits de dégradation de l'amitriptyline HCl** | **SPECIFI-CATIONS** | **RESULTATS** | | | |
|---|---|---|---|---|---|
| | | **T0** | **T1 mois** | **T3 mois** | **T6 mois** |
| Cyclobenzaprine | ≤ 0,1% | < limite de détection | < limite de détection | < limite de détection | < limite de détection |
| Dibenzosuberone | ≤ 0,1% | Non détectée | Non détectée | Non détectée | Non détectée |
| Impuretés inconnues | Reportée si ≤ 0,1%, | Non détectée | Non détectée | Non détectée | Impureté inconnue 1 RRT 0,38 min < 0,1%; |
| | Aucune < 0,2% | | | | Impureté inconnue 2 RRT 0,45 min < 0,1% |
| Total Impuretés | ≤ 1 % | N/A | N/A | N/A | < 0,1% |

Aucune synérèse n'a été observé pour la composition C sous forme de gel aqueux selon l'invention après 6 mois à 40°C.

On observe également aucune variation majeure sur chacun des tests réalisés.

On peut ainsi noter la bonne stabilité physico-chimique des compositions sous forme de gel aqueux selon l'invention.

Il a également été observé que la composition C sous forme de gel aqueux selon l'invention est particulièrement efficace dans le traitement des douleurs neuropathiques du membre fantôme.

## Revendications

1. Composition pharmaceutique sous forme de gel aqueux comprenant au moins de l'amitriptyline et/ou l'un de ses sels pharmaceutiquement acceptable, dont la teneur totale en amitriptyline et/ou en l'un de ses sels pharmaceutiquement acceptable est comprise entre 10 et 30% en poids par rapport au poids total de la composition, pour son utilisation par voie topique dans le traitement des douleurs neuropathiques du membre fantôme.

2. Composition selon la revendication précédente, pour son utilisation par voie cutanée dans le traitement des douleurs neuropathiques du membre fantôme.

3. Composition selon l'une quelconque des revendications précédentes, pour son utilisation à titre préventif ou à titre curatif.

4. Composition selon l'une quelconque des revendications précédentes, **caractérisée en ce que** la teneur totale en amitriptyline et/ou en l'un de ses sels pharmaceutiquement acceptable est comprise entre 10 et 25% en poids, de préférence entre 10 et 20% en poids, plus préférentiellement encore entre 10 et 15% en poids, par rapport au poids total de la composition.

5. Composition selon l'une quelconque des revendications précédentes, **caractérisée en ce qu'**elle comprend au moins un polymère cellulosique ; de préférence choisis parmi les éthers de cellulose ; plus préférentiellement parmi les éthers de cellulose non-ioniques ; plus préférentiellement encore parmi (a) les (Ci-C₄)alkylcelluloses telles que les méthylcelluloses et les éthylcelluloses, (b) les (poly)hydroxy(C₁-C₄)alkylcelluloses telles que les hydroxyméthylcelluloses, les hydroxyéthylcelluloses et les hydroxypropylcelluloses, (c) les celluloses mixtes (poly)hydroxy(C₁-C₄)alkyl-(C₁-C₄)alkylcelluloses telles que les hydroxypropyl-méthylcelluloses, les hydroxyéthyl-méthylcelluloses, les hydroxypropyl-éthylcelluloses, les hydroxyéthyl-éthylcelluloses et les hydroxybutyl-méthylcelluloses, et (d) leurs mélanges ; mieux la composition comprend au moins une (poly)hydroxy(C₁-C₄)alkylcellulose telles que les hydroxyméthylcelluloses, les hydroxyéthylcelluloses et les hydroxypropylcelluloses ; et encore mieux la composition comprend au moins l'hydroxyéthylcellulose.

6. Composition selon l'une quelconque des revendications précédentes, **caractérisée en ce qu'**elle comprend au moins un polyol en C₂-C₈ ; de préférence choisis parmi les polyols en C₃-C₆, l'éthylène glycol, et leurs mélanges ; plus préférentiellement parmi le propylène glycol, le 1,3-propanediol, le 1,3-butylène glycol, le pentane-1,2-diol, le dipropylène glycol, l'hexylène glycol, le pentylène glycol, le glycérol, l'éthylène glycol, et un mélange de ces composés ; plus préférentiellement encore la composition comprend au moins le propylène glycol.

7. Composition selon l'une quelconque des revendications précédentes, **caractérisée en ce que** la viscosité, à une température de 20°C et à pression atmosphérique, est comprise entre 400 et 2500 mPa.s ; de préférence entre 600 et 2000 mPa.s ; et plus préférentiellement entre 800 et 1500 mPa.s.

8. Composition selon l'une quelconque des revendications précédentes, **caractérisée en ce que** la teneur en eau est supérieure ou égale à 65% en poids, de préférence comprise entre 65 et 90% en poids ; plus préférentiellement entre 70 et 90% en poids, plus préférentiellement encore entre 75 et 85% en poids, par rapport au poids total de la composition.

9. Composition selon l'une quelconque des revendications précédentes, **caractérisée en ce qu'**elle est exempte de corps gras.

10. Composition selon l'une quelconque des revendications précédentes, **caractérisée en ce que** le pH est compris entre 3 et 8, de préférence entre 4 et 7, et plus préférentiellement entre 5 et 6.

11. Composition selon l'une quelconque des revendications précédentes, comprenant :
(i) de 10 à 30% en poids d'amitriptyline ou de l'un de ses sels pharmaceutiquement acceptable, de préférence de 10 à 20 % en poids, plus préférentiellement plus de 10 à 15 % en poids, par rapport au poids total de la composition ;
(ii) de 0,1 à 10% en poids d'au moins un polymère cellulosique, par rapport au poids total de la composition ;
(iii) de 0,1 à 15% en poids d'au moins un polyol en C₂-C₈, par rapport au poids total de la composition ; et
(iv) de l'eau, de préférence en une teneur allant de 65 à 90% en poids par rapport au poids total de la composition.
